# EUROPEAN PATENT SPECIFICATION

(11) **EP 2 153 810 B1**
(45) Date of publication and mention of the grant of the patent: **17.10.2012**
(21) Application number: 08764583.4
(22) Date of filing: 23.05.2008
(51) Int. Cl.: A61F 13/15, A61F 13/515, B29C 65/02

(54) **ABSORPTIVE ARTICLE**
SAUGFÄHIGER ARTIKEL
ARTICLE ABSORBANT

(30) Priority: 24.05.2007 JP 2007138221
(43) Date of publication of application: 17.02.2010
(73) Proprietor: Uni-charm Corporation, Ehime 799-0111 (JP)
(72) Inventor: NODA, Yuki, Kanonji-shi Kagawa 769-1602 (JP); NISHIKAWA, Kumiko, Kanonji-shi Kagawa 769-1602 (JP); KURODA, Kenichiro, Kanonji-shi Kagawa 769-1602 (JP)
(74) Representative: Sperling, Rüdiger
(86) International application number: PCT/JP2008/059537
(87) International publication number: WO 2008/146737

(56) References cited:
- CA-A1- 2 561 457
- GB-A- 1 332 037
- JP-A- 08 322 879
- JP-A- 11 056 902
- JP-A- 2001 129 019
- JP-A- 2002 515 805
- JP-A- 2004 008 592
- JP-A- 2007 216 031
- US-A- 5 626 574
- US-A1- 2003 065 295
- US-A1- 2006 089 616
- US-B1- 6 717 028

## Description

### TECHNICAL FIELD

The present invention relates to an absorbent article.

### BACKGROUND ART

Absorbent articles such as a sanitary napkin or a sheet for absorbing vaginal discharge have been widely known as absorbents for absorbing liquids such as the discharged matter discharged from the vaginal opening of females. In general, absorbent articles include liquid retentive absorbent layers, liquid-permeable top sheets and liquid-impermeable back surface sheets, in which the perimeter thereof is sealed with the heat embossment processing or the like. The sealed portion thus sealed is required to maintain the strength so as to prevent itself from breaking while in use, and also required not to make the wearer uncomfortable in use.

Conventionally, in order to seal the perimeter of the absorbent article, a method has been widely known in which the sealed portion is formed in a planar shape by heat embossing process or the like. However, the sealed portion formed in this way is hardened due to the compression in corresponding regions by the embossing process, as a result of which there are cases where the wearer feels uncomfortable while using the absorbent article. Furthermore, the hardening of the corresponding regions makes it hard for the absorbent article to deform along the curve of the wearer's body, thereby causing a gap between the body and the absorbent article, also resulting in leakage.

Accordingly, an invention has been disclosed, in which instead of the embossing process in a planar shape, a plurality of small and substantially square embossed portions are formed, as a result of which the number of folding-starting points is increased, which are formed in non-embossed portions between adjacent embossed portions in the sealed portion, thereby making it easy for the absorbent article to deform along the curve of the wearer's body (for example, Japanese Unexamined Patent Application Publication No. 2001-129019). Moreover, the embossed portions are always arranged to the adjacent embossed portions in serial, such that an area per embossed portion is larger in portions with greater weight or thickness, and an area per embossed portion is smaller in the other portions.

US2003/0065295A1 discloses absorbent articles comprising bonded composites wherein the bonded composite has first and second thin-section elements bonded to each other, at least in part by bond elements and at least in part by adherent material. The adherent material is disposed between the first and second thin-section elements proximate and about the bond elements. The bond patterns are arranged and configured to direct stresses imposed on the bond pattern, inwardly into the interior of the bond pattern.

### DISCLOSURE OF THE INVENTION

### Problems to be Solved by the Invention

However, as for the absorbent article disclosed in Japanese Unexamined Patent Application Publication No. 2001-129019, the embossed portions with greater weight are hardened due to the heat, as a result of which the wearer feels uncomfortable while using the absorbent article. Moreover, if an area per embossed portion becomes large, the distance between folding-starting points that are formed in non-embossed portions is widened, thereby decreasing the flexibility in the perimeter. Since this makes it harder for the absorbent articles to deform along the curve of the body, it is apprehended that a gap is caused, resulting in leakage.

It is an object of the present invention to provide an absorbent article which has superior feeling and which is hard to leak, by maintaining the flexibility in the sealed portion in the perimeter of the absorbent article.

### Means for Solving the Problems

In a first aspect of an absorbent article of the present invention, an elongated absorbent article is provided, including a top sheet portion having a sheet, at least a part of which is liquid-permeable; a back surface sheet portion having a liquid-impermeable sheet; and a liquid retentive absorbent core arranged between the top sheet portion and the back surface sheet portion, in which a joined region, which joins the back surface sheet portion and the top sheet portion, is formed in an outer perimeter of the absorbent article, in which a plurality of pressure-bonding parts are formed in all or part of the joined region, and in which each of the plurality of pressure-bonding parts is formed so that the length in a first direction is longer than the length in a second direction that is orthogonal to the first direction.

In a second aspect of the absorbent article as described in the first aspect of the present invention, the absorbent article is provided, in which, in each of the plurality of pressure-bonding parts, the length in the first direction is 1.7 to 20 in relation to the length in the second direction.

In a third aspect of the absorbent article as described in any one of the first or second aspect of the present invention, the absorbent article is provided, in which, in the plurality of pressure-bonding parts, the length in the first direction is 1.0 to 10 mm, and the length in the second direction is 0.5 to 2.0 mm.

In a fourth aspect of the absorbent article as described in any one of the first to third aspects of the present invention, the absorbent article is provided, in which the plurality of pressure-bonding parts have first pressure-bonding parts and second pressure-bonding parts, and in which the second pressure-bonding parts are formed in the second direction in the first pressure-bonding parts.

In a fifth aspect of the absorbent article as described in the fourth aspect of the present invention, the absorbent article is provided, in which the distance between the first pressure-bonding part and the second pressure-bonding part in the second direction is 0.3 to 4.0 relative to the length of the first pressure-bonding part in the second direction.

In a sixth aspect of the absorbent article as described in any one of the fourth or fifth aspect of the present invention, the absorbent article is provided, in which the distance between the first pressure-bonding part and the second pressure-bonding part in the second direction is 1.0 to 5.0 mm.

In a seventh aspect of the absorbent article as described in any one of the fourth to sixth aspects of the present invention, the absorbent article is provided, in which all or part of the plurality of pressure-bonding parts formed at the ends of a longitudinal direction of the absorbent article is formed to be substantially orthogonal to the longitudinal direction.

In an eighth aspect of the absorbent article as described in any one of the fourth to seventh aspects of the present invention, the absorbent article is provided, in which, in all or part of the plurality of pressure-bonding parts, the first direction is formed to be substantially parallel to the outer perimeter.

In a ninth aspect of the absorbent article as described in any one of the fourth to eighth aspects of the present invention, the absorbent article is provided, in which the second pressure-bonding part is formed to intersect a straight line extending in the second direction from one end of the first direction of the first pressure-bonding part, and not to intersect a straight line extending in the second direction from the other end of the first direction.

In a tenth aspect of the absorbent article as described in any one of the fourth to ninth aspects of the present invention, the absorbent article is provided, in which the plurality of first pressure-bonding parts and second pressure-bonding parts are respectively formed, in which the plurality of first pressure-bonding parts and the plurality of second pressure-bonding parts are formed in such a way that the first direction is substantially parallel to the outer perimeter, and are formed in series in the outer perimeter direction, and in which non-pressure-bonding regions formed between the plurality of first pressure-bonding parts and the plurality of second pressure-bonding parts are formed to be substantially parallel to the outer perimeter direction, and are formed to extend in the outer perimeter direction.

In an eleventh aspect of the absorbent article as described in any one of the first to tenth aspects of the present invention, the absorbent article is provided, in which each of the plurality of pressure-bonding parts is substantially the same shape.

In a twelfth aspect of the absorbent article as described in the eleventh aspect of the present invention, the absorbent article is provided, in which the joined region has a plurality of auxiliary pressure-bonding parts which are shaped differently from the plurality of pressure-bonding parts, and in which the plurality of pressure-bonding parts and the plurality of auxiliary pressure-bonding parts are formed alternately in the outer perimeter direction.

In a thirteenth aspect of the absorbent article as described in any one of the first to twelfth aspects of the present invention, the absorbent article is provided, in which, in a predetermined position in the outer perimeter, the joined region is not formed, or a region is formed in which the number of the plurality of pressure-bonding parts is small.

### Effects of the Invention

According to the present invention, it is possible to provide an absorbent article which has superior feeling and which is hard to leak, by maintaining the flexibility of the sealed portion in the perimeter of the absorbent article.

### BRIEF DESCRIPTION OF THE DRAWINGS

Fig. 1A is a plan view for showing a first embodiment of the absorbent article according to the present invention;
Fig. 1B is an enlarged view for showing a shape of a first pressure-bonding part in the first embodiment;
Fig. 2 is a cross-sectional view of Fig. 1A taken along the line Z-Z;
Fig. 3A is a plan view for showing a second embodiment of the absorbent article according to the present invention;
Fig. 3B is an enlarged view for showing a shape of the pressure-bonding part in the second embodiment;
Fig.4 is a plan view for showing a third embodiment of the absorbent article according to the present invention;
Fig.5 is a plan view for showing a fourth embodiment of the absorbent article according to the present invention;
Fig. 6A is a plan view for showing a fifth embodiment of the absorbent article according to the present invention; and
Fig. 6B is an enlarged view for showing a shape of the pressure-bonding part in the fifth embodiment.

### PREFERRED MODE FOR CARRYING OUT THE INVENTION

The preferred embodiments of the present invention will be hereinafter explained with reference to the drawings. Note that the embodiments are intended to show preferred embodiments, and are not limited to the drawings and the descriptions.

### 1. First Embodiment

### 1-1. Overall Configuration of Absorbent Article

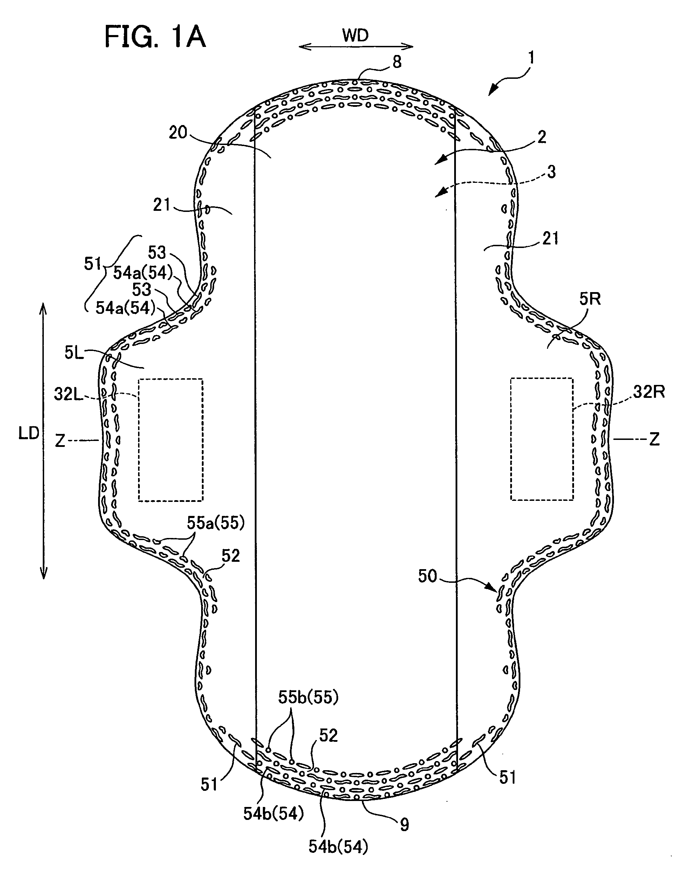

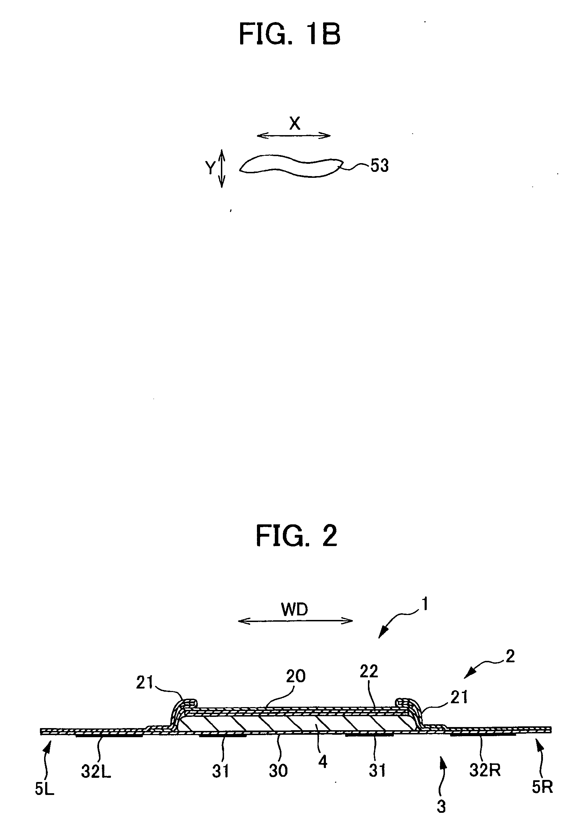

As shown in Fig. 1A and Fig. 2, an absorbent article 1 according to the present invention is formed in an elongated-shape. The absorbent article 1 includes a top sheet portion 2 which is at least partly liquid-permeable, a back surface sheet 3 portion which serves as a leakproof layer, and an absorbent core 4 which absorbs and holds liquids such as the discharged matter.

The top sheet portion 2 and a back surface sheet 30 to be described later that constitutes the back surface sheet portion 3 are joined along the outer perimeter by the heat embossing process, thereby forming a sealed portion 50 that is a joined region.

As shown in Fig. 1A and Fig. 2, the top sheet portion 2 is constituted with a top sheet 20 which is arranged in the middle of the width direction WD of the absorbent article 1, side sheets 21 and 21 which are arranged along the longitudinal direction LD on both sides of the width direction WD of the top sheet 20, and a second sheet 22 which is arranged between the top sheet 20 and the absorbent core 4.

Top sheet 20 is a liquid-permeable sheet, is arranged in such a way to cover the absorbent core 4 and the second sheet 22, and is a sheet which, in the absorbent article 1, contacts with the wearer's body. For the top sheet 20, it is possible to use a known liquid-permeable sheet used in a general absorbent article. For example, it is possible to use a non-woven fabric formed by thermoplastic fibers or the like. Liquids such as the discharged matter are then permeated into the side of the absorbent core 4.

The side sheets 21 and 21 are arranged along the longitudinal direction LD of the absorbent article 1, on both sides of the width direction WD of the top sheet 20. Parts of the side sheets 21 and 21 in the longitudinal direction LD of the absorbent article 1 protrude toward the width direction WD, and respectively form wings 5L and 5R together with the back surface sheet 30 to be described later.

The side sheets 21 and 21 are joined to the top sheet 20 with a hot melt adhesive and the embossed parts (not shown) formed at substantially equal distance along the longitudinal direction LD, on both sides of the width direction WD of the top sheet 20. In detail, as shown in Fig. 2, the side sheets 21 and 21 are folded back at the portions where the side sheets overlap the top sheet 20 and the absorbent core 4, such that the inward regions located in the side sheets inward to the width direction WD of the absorbent article 1 serve as the folding edges. The side sheets 21 and 21 are joined to the top sheet 20, of which the outer edges of the absorbent article 1 in the width direction WD extend toward the outside of the absorbent core 4.

The second sheet 22 is arranged between the top sheet 20 and the absorbent core 4. The second sheet 22 is arranged in a state where the liquid-permeable sheet is folded in two. Though the second sheet 22 can be formed from the materials similar to those of the top sheet 20, it is preferable to be formed such that the density is higher than that of the top sheet 20. The density of the second sheet 22 is higher than that of the top sheet 20, thereby making it possible to increase the liquid-transport property from the top sheet 20.

The length of the top sheet 20 and the side sheets 21 in the longitudinal direction LD is formed to be greater than the length of the absorbent core 4 in the longitudinal direction LD. Moreover, the length of the top sheet 20 in width direction WD is formed to be the same as, or greater than, the length of the absorbent core 4 in width direction WD.

The back surface sheet portion 3 constitutes the leakproof layer in the absorbent article 1. The back surface sheet portion 3 includes a liquid-impermeable back surface sheet 30, dislocation-preventing portions 31 that fix the absorbent article 1, and wing-side dislocation-preventing portions 32L and 32R. The back surface sheet 30 is formed, for example, of a liquid-impermeable sheet such as a plastic film, and controls so that liquids such as the discharged matter held in the absorbent core 4 do not adhere to the underwear. The dislocation-preventing portions 31 are arranged, in the back surface sheet 30, at the side opposite to the side where the absorbent core 4 is arranged, and fix the absorbent article 1 by adhering to the underwear. Moreover, as for the wing-side dislocation-preventing portions 32L and 32R, after the absorbent article 1 is arranged at the crotch area of the underwear, the wings 5L and 5R are folded back to the side of the underwear, thereby fixing the absorbent article 1 to the underwear.

The length of the back surface sheet 30 in the longitudinal direction LD is formed to be greater than the length of the absorbent core 4 in the longitudinal direction LD. Moreover, as described above, the length of the top sheet 20 and the side sheets 21 constituting the top sheet portion 2, in the longitudinal direction LD, is formed to be greater than the length of the absorbent core 4 in the longitudinal direction LD. In other words, a front edge 8 and a rear edge 9 of the absorbent article 1 are in a state where the top sheet portion 2 and the back surface sheet 30 extend outward to the longitudinal direction LD, without the absorbent core 4 being arranged. The sealed portion 50 is formed along the outer perimeter of the absorbent article 1 in portions where this formation is performed in the top sheet portion 2 and the back surface sheet 30.

The length of the absorbent article 1 in the longitudinal direction LD is between 100 and 500 mm inclusive, and preferably between 150 and 350 mm inclusive. Moreover, the length in the width direction WD is between 30 and 200 mm inclusive, and preferably between 40 and 180 mm inclusive.

Absorbent core 4 is arranged by the width direction WD center of absorbent article 1, liquid such as discharged matter excreted by wearer's body is taken in, and it is held. The size of the absorbent core 4 is formed so that the lengths in the longitudinal direction LD and the width direction WD are smaller than those of the top sheet portion 2 to be described later.

The wings 5L and 5R are formed to protrude outward to the width direction WD, on the respective sides of the absorbent article 1 in the width direction WD. The wings 5L and 5R are formed of the side sheets 21 and a part of the back surface sheet 30, on both sides of the top sheet 20 in the width direction WD.

The sealed portion 50 is formed along the outer perimeter of the absorbent article 1. It is preferable that the absorbent article 1, including the wings 5L and 5R, as a whole be formed with a curved outer periphery.

### 1-2. Sealed Portion

As shown in Fig. 1A, the sealed portion 50 is formed along the outer perimeter of the absorbent article 1. The sealed portion 50 is formed along the outer perimeter of the absorbent article 1, continuously or intermittently for the entire circumference of the absorbent article 1.

The sealed portion 50 includes a plurality of pressure-bonding parts 51 and non-pressure-bonding parts 52 formed by the heat embossing process. In the first embodiment, the sealed portion 50 includes a plurality of pressure-bonding parts 51, auxiliary pressure-bonding parts 55 of which the shape is different from that of the plurality of pressure-bonding parts 51, and the non-pressure-bonding parts 52.

The shape of the pressure-bonding parts 51 is formed in such a way that the length in the first direction X in the pressure-bonding parts 51 is longer than the length in the second direction Y that is orthogonal to the first direction X. Here, the length of the pressure-bonding part 51 in the first direction X refers to the length of a line segment between both ends of the pressure-bonding part 51 in the first direction X. Moreover, in a case where straight lines (straight lines extending in the second direction Y) are drawn orthogonally to the first direction X of the pressure-bonding part 51, and the periphery of the pressure-bonding part 51 and the straight lines extending in the second direction Y intersect, the length of the longest line between two intersection points is the length of the pressure-bonding part 51 in the second direction Y.

Moreover, the pressure-bonding parts 51 include the first pressure-bonding parts 53 as well as the second pressure-bonding parts 54 formed to the side of the second direction Y. As shown in Figs. 1A and 1B, the first pressure-bonding parts 53 are formed in such a way that the first direction X is substantially parallel to the direction along the outer perimeter of the absorbent article 1, respectively at predetermined intervals for substantially entire circumference of the absorbent article 1. Moreover, the second pressure-bonding parts 54 are formed inward the width direction WD of the first pressure-bonding parts 53, at a predetermined distance from the first pressure-bonding parts 53, in such a way that the first direction X is substantially parallel to the direction along the outer perimeter of the absorbent article 1, respectively at predetermined intervals for substantially entire circumference of the absorbent article 1. That is to say, a broken-line shape formed by the plurality of the first pressure-bonding parts 53, and a broken-line shape formed by the plurality of the second pressure-bonding parts 54 are formed in two lines for substantially entire circumference of the absorbent article 1.

The non-pressure-bonding parts 52 formed between the first pressure-bonding parts 53 and the second pressure-bonding parts 54 are substantially parallel to the direction along the outer perimeter of the absorbent article 1, and are formed in succession in the direction along the outer perimeter. Moreover, the second pressure-bonding part 54 is arranged in such a way that the second pressure-bonding part 54 intersects a straight line extending in the second direction Y from one end of the first pressure-bonding part 53 in the first direction X, but do not intersect a straight line extending in the second direction Y from the other end of the first pressure-bonding part 53 in the first direction X. That is to say, the first pressure-bonding parts 53 and the second pressure-bonding parts 54 are arranged in a staggered manner.

In the first embodiment, all of the first pressure-bonding parts 53 have the same shape. Moreover, the second pressure-bonding parts 54a, which are formed on both sides of the longitudinal direction LD of the absorbent article 1, have the same shape as the first pressure-bonding parts 53.

As shown in Fig. 1B, the shape of the first pressure-bonding parts 53 and the second pressure-bonding parts 54a, which are formed on both sides of the longitudinal direction LD of the absorbent article 1, is formed in such a way that the length in the first direction X is greater than the length in the second direction Y. Moreover, it is possible to form, with a curve, each or part of edges that form the second pressure-bonding parts 54a formed on both sides of the longitudinal direction LD of the first pressure-bonding parts 53 and the absorbent article 1. In the first embodiment, the outer circumference of the second pressure-bonding parts 54a, which are formed on both sides of the longitudinal direction LD of the first pressure-bonding parts 53 and the absorbent article 1, is formed with a curve. The first pressure-bonding parts 53 has a shape which is meandering as a whole from one end to the other end of the first direction X, with the both ends being apexes of the shape. The second pressure-bonding parts 54a, which are formed on both sides of the longitudinal direction LD of the first pressure-bonding parts 53 and the absorbent article 1, have a shape in which both edges extending in the first direction X meander substantially in parallel to each other, the edges intersecting at both ends of the first direction X to form apexes.

In the sealed portion 50 in the vicinity of the front edge 8 and the rear edge 9 of the absorbent article 1, the shape of the second pressure-bonding parts 54b is different from the shape of the second pressure-bonding parts 54a formed on both sides of the longitudinal direction LD of the absorbent article 1. The shape of the second pressure-bonding parts 54b is formed with a curve, in which edges extending in the first direction X bulge outward at a substantial center of the first direction X, with both ends of the first direction X being apexes.

Moreover, in the sealed portion 50 in the vicinity of the front edge 8 and the rear edge 9 of the absorbent article 1, the second pressure-bonding parts 54 are formed inward the absorbent article 1, and the first pressure-bonding parts 53 are formed respectively at predetermined intervals in such a way that the first direction X of the first pressure-bonding parts 53 is substantially in parallel to the direction along the outer perimeter of the absorbent article 1. In addition, further inward the absorbent article 1, the second pressure-bonding parts 54b are formed respectively at predetermined intervals in such a way that the first direction X is substantially in parallel to a direction along the outer perimeter of the absorbent article 1. That is to say, in the sealed portion 50 in the vicinity of the front edge 8 and the rear edge 9 of the absorbent article 1, a broken-line shape formed of the first pressure-bonding parts 53 and a broken-line shape formed of the second pressure-bonding parts 54b are alternately formed in four lines in total.

In the first embodiment, as described above, the sealed portion 50 has the auxiliary pressure-bonding parts 55 with the shape that is different from the shape of the plurality of pressure-bonding parts 51 (the first pressure-bonding parts 53 and the second pressure-bonding parts 54), and the auxiliary pressure-bonding parts 55 are respectively formed between the two second pressure-bonding parts 54 and 54 that are adjacent to each other along the direction of the outer perimeter of the absorbent article 1, and between the two first pressure-bonding parts 53 and 53 that are adjacent to each other in the direction of the outer perimeter of the absorbent article 1. In other words, the plurality of pressure-bonding parts 51 and the plurality of auxiliary pressure-bonding parts 55 are alternately formed along the direction of the outer perimeter of the absorbent article 1.

The auxiliary pressure-bonding parts 55 assist the joining of the top sheet portion 2 and the back surface sheet 30 by the first pressure-bonding parts 53 and the second pressure-bonding parts 54. The auxiliary pressure-bonding parts 55 are different from the first pressure-bonding parts 53 and the second pressure-bonding parts 54 in that the length extending in the first direction X and the length extending in the second direction Y may be the same, and that any one of the length extending in the first direction X and the length extending in the second direction Y may be greater than the other. In the first embodiment, the auxiliary pressure-bonding parts 55 include heart-shaped auxiliary pressure-bonding parts 55a that are formed on both sides of the longitudinal direction LD of the absorbent article 1, and substantially circular auxiliary pressure-bonding parts 55b that are formed in the vicinity of the front edge 8 and the rear edge 9 of the absorbent article 1.

Thus, in the first embodiment, on both sides of the longitudinal direction LD of the absorbent article 1, a broken-line shape, in which the first pressure-bonding parts 53 and the heart-shaped auxiliary pressure-bonding parts 55a are alternately arranged, is formed to the side of the outer perimeter thereof, and a broken-line shape, in which the second pressure-bonding parts 54a and the heart-shaped auxiliary pressure-bonding parts 55a are alternately arranged, is formed inside thereof. Moreover, in the sealed portion 50 in the vicinity of the front edge 8 and the rear edge 9 of the absorbent article 1, a broken-line shape, in which the first pressure-bonding parts 53 and the substantially circular auxiliary pressure-bonding parts 55b are alternately arranged, and a broken-line shape, in which the second pressure-bonding parts 54b and the substantially circular auxiliary pressure-bonding parts 55b are alternately arranged, are alternately formed in four lines from the outer perimeter.

In the boundary between the front edge 8 as well as the rear edge 9 in the absorbent article 1 and the side edges along the longitudinal direction LD of the absorbent article 1, a region is formed in which the number of the pressure-bonding parts 51 is small. In the first embodiment, in the side edges to the longitudinal direction LD in the vicinity of the boundary between the front edge 8 as well as the rear edge 9 and the side edges to the longitudinal direction LD, regions are formed in which the pressure-bonding parts 51 are formed in only one line.

The length extending in the first direction X in the first pressure-bonding parts 53 and the second pressure-bonding parts 54 is between 1.0 and 10 mm inclusive, and preferably between 2.0 and 7.0 mm inclusive. When the length extending in the first direction X in the first pressure-bonding parts 53 and the second pressure-bonding parts 54 is greater than 10 mm, it is apprehended that the flexibility of the sealed portion 50 is reduced, and the rigidities differ within the region occupied by the first pressure-bonding parts 53 and the second pressure-bonding parts 54, or the rigidities differ in the boundary between the first pressure-bonding parts 53 as well as the second pressure-bonding parts 54 and the non-pressure-bonding parts 52 in the vicinity thereof, resulting in edge-folding starting from such a region, thereby making the wearer uncomfortable in use and causing leakage of bodily fluids. Moreover, since the first pressure-bonding parts 53 and the second pressure-bonding parts 54 are formed by the heat embossing process, the embossed portions have been melted or thinned. As a result, when a part of the sealed portion 50 is pulled during use of the absorbent article 1, there is a possibility that this becomes a starting point of tearing.

The length extending in the second direction Y in the first pressure-bonding parts 53 and the second pressure-bonding parts 54 is between 0.5 and 2.0 mm inclusive, and preferably between 0.7 and 1.5 mm inclusive. In cases where the length extending in the second direction Y is smaller than 0.5 mm, the first pressure-bonding parts 53 and the second pressure-bonding parts 54 may serve as a so-called perforated line, thereby the material may be cut in the middle, depending on the arrangement pattern of the first pressure-bonding parts 53 and the second pressure-bonding parts 54. Moreover, in cases where the length extending in the second direction Y in the first pressure-bonding parts 53 and the second pressure-bonding parts 54 is greater than 1.5 mm, it is apprehended that the area compressed by the first pressure-bonding parts 53 and the second pressure-bonding parts 54 is increased, and the feeling is reduced.

Furthermore, the ratio of the length extending in the first direction X and the length extending in the second direction Y is between 1.7 and 20 inclusive, and preferably between 3 and 7 inclusive. In cases where the ratio of the length extending in the first direction X and the length extending in the second direction Y is smaller than 1.7, it may become difficult to obtain an effect of increasing the feeling while maintaining the strength of the pressure-bonding parts 51 by differentiating the length in the first direction X and the length in the second direction Y of the pressure-bonding parts 51. In cases where the ratio of the length extending in the first direction X and the length extending in the second direction Y is greater than 20, the length of the first direction X becomes longer, as a result of which the edge-folding as described above may tend to occur, and the top sheet 20 may tend to tear when the top sheet 20 is pulled.

The distance between the two first pressure-bonding parts 53 adjacent to each other in the first direction X is preferably between 0.08 and 2.5 inclusive in relation to the length of the first pressure-bonding part 53 in the first direction X. Moreover, the distance between the two first pressure-bonding parts 53 adjacent to each other in the first direction X is preferably between 0.8 and 5.0 mm inclusive, and more preferably between 1.0 and 2.5 mm inclusive.

In cases where the distance between the two first pressure-bonding parts 53 adjacent to each other in the first direction X is smaller than 0.8 mm, the space between the first pressure-bonding parts 51 is narrowed, and the rigidity of the non-pressure-bonding parts 52 is increased, thereby it is apprehended that the feeling of the absorbent article 1 by the wearer in use may be harmed. In cases where the distance between the two first pressure-bonding parts 53 adjacent to each other is greater than 5.0 mm, the space between the pressure-bonding parts is widened, thereby it is apprehended that bodily fluids may leak from the non-pressure-bonding parts 52. Moreover, since the region-ratio of the non-pressure-bonding parts 52 to the pressure-bonding parts 51 in the sealed portion 50 becomes greater, it is apprehended that openings may occur in the sealed portion 50.

The distance between the first pressure-bonding part 53 and the second pressure-bonding part 54 that are adjacent to each other in the second direction Y is preferably between 0.5 to 1.5 inclusive in relation to the length of the first pressure-bonding part 53 in the second direction Y. Moreover, the distance between the first pressure-bonding part 53 and the second pressure-bonding part 54 in the second direction Y is preferably between 1.0 to 5.0 mm inclusive, and more preferably between 1.2 and 2.5 mm inclusive.

For example, a case is exemplified, in which only the first pressure-bonding parts 53 are formed in the sealed portion 50, the ratio of the length extending in the first direction X and the length extending in the second direction Y is 1.7, and the length of the short sides to the longitudinal direction LD is 0.5 mm. In this case, if the distance between the first pressure-bonding parts 53 adjacent to each other is 0.5 mm, the number of the first pressure-bonding parts that can be arranged increases by 1.2 times. Moreover, in this case, the length of the long sides, at the time when the length extending in the second direction Y is 0.5 mm, is (0.65×0.65)/0.5=0.845 mm.

In the first embodiment, it is possible to arrange the pressure-bonding parts 51 adjacent to each other with a greater distance. This makes it possible to maintain the flexibility of the sealed portion 50 in the absorbent article 1, without harming the feeling and without causing uncomfortability for the wearer.

Moreover, in cases where the first pressure-bonding parts 53 and the second pressure-bonding parts 54 are arranged along the perimeter of the absorbent article 1, it is possible to arrange the pressure-bonding parts adjacent to each other in the second direction Y with a smaller distance. As a result, the folding-starting points are increased, which are formed by the non-pressure-bonding parts 52 that are formed between the pressure-bonding parts adjacent to each other in the second direction Y, thereby making it possible to deform the article along the curve of the wearer's body. This makes it possible to prevent the leakage arising from a gap between the article and the wearer's body.

For example, it is assumed that only the rectangular pressure-bonding parts are formed in the pressure-bonding parts 51. It is also assumed that an area per one rectangular pressure-bonding part is 2.25 mm². In cases where the length extending in the first direction X is different from the length extending in the second direction Y in the rectangular pressure-bonding parts, and the rectangular pressure-bonding parts are formed with the same area, for example, the length extending in the first direction is 2.25 mm, and the length extending in the second direction Y is 1.0 mm, as for the rectangular pressure-bonding parts. Moreover, if the distance between the first pressure-bonding parts adjacent to each other in the second direction Y is 0.5 mm that is the same as described above, the pitch in the first direction X is 2.75 mm, and the pitch in the second direction Y is 1.5 mm.

Here, the pitch in the first direction X refers to the distance in the first direction X between the centers of a pair of the first pressure-bonding parts 53 adjacent to each other in the first direction X. Moreover, the pitch in the second direction Y refers to the distance between the centers of a pair of the first pressure-bonding parts 53 adjacent to each other in second direction Y.

On the other hand, in cases where the pressure-bonding parts are formed in which the length extending in the first direction X is the same as the length extending in the second direction Y, each side needs to be 1.5 mm with the area that is the same as the above. Moreover, in cases where the distance between the pressure-bonding parts adjacent to each other is 0.5 mm, the pitch between the pressure-bonding parts adjacent to each other is 2.0 mm both in the first direction X and the second direction Y.

Thus, the pitch in the second direction Y in the pressure-bonding parts, in which the length extending in the first direction X is different from the length extending in the second direction Y, is smaller than the length in the second direction Y in the pressure-bonding parts, in which the length extending in the first direction X is the same as the length extending in the second direction Y. Specifically, the pitch in the second direction Y is 1.5 mm. Thus, in cases where the area of the pressure-bonding parts per unit area is maintained, it is possible to widen the pitch in the second direction Y by forming the pressure-bonding parts in which the length extending in the first direction X is different from the length extending in the second direction Y. This makes it possible to widen the region in which the pressure-bonding parts are not formed, and to maintain the feeling and the softness.

Moreover, it is possible to increase the number of the pressure-bonding parts to be arranged in the second direction Y, in cases of the pressure-bonding parts in which the length extending in the first direction X is different from the length extending in the second direction Y. Since the area of the pressure-bonding parts per unit area can be easily increased, it is possible to increase the joining strength. Moreover, since the number of the folding-starting points is increased, it is possible to maintain the flexibility in the sealed portion 50.

In the first embodiment, the pressure-bonding parts 51 are arranged along the outer perimeter of the absorbent article 1. As a result, it is possible to increase the resistance to the bending to the width direction WD, particularly in the front edge 8 or the rear edge 9. Moreover, the front edge 8 and the rear edge 9 are formed with a curve, and the pressure-bonding parts 51 are also formed along this curve. As a result, in the front edge 8 and the rear edge 9, the pressure-bonding parts 51 are not arranged on any of the straight lines in the longitudinal direction LD and in the width direction WD of the absorbent article 1, thereby making the article hard to fold.

In the first embodiment, the shape of pressure-bonding parts 51 is formed so that the outer perimeter thereof is a curve. Thus, the pressure-bonding parts 51 are not arranged to form a straight line, as a result of which the folding-starting points are dispersed, thereby making it possible to prevent the ends of the absorbent article 1 from breaking at the time of wearing.

In the first embodiment, the second pressure-bonding parts 54 are arranged in such a way that the second pressure-bonding part 54 intersects the straight line extending in the second direction Y from one end of the first direction X of the first pressure-bonding parts 53, but does not intersect the straight line extending in the second direction Y from the other end of the first direction X of the first pressure-bonding parts 53. That is to say, the first pressure-bonding parts 53 and the second pressure-bonding parts 54 are arranged in a staggered manner. As a result, the non-pressure-bonding parts 52 are not formed in series in the second direction Y, in the longitudinal direction LD or in the width direction WD of the absorbent article 1. Particularly in the front edge 8 and the rear edge 9 of the absorbent article 1, the non-pressure-bonding parts 52 are not formed in series in the longitudinal direction LD of absorbent article 1, thereby making it hard to fold in the width direction WD. This makes it possible to maintain the absorbent article 1 in a spread state, thereby suppressing the twisting of the absorbent article 1 while in use

According to the first embodiment, since the first pressure-bonding parts 53 are formed in which the lengths respectively extending in the first direction X and the second direction Y are different, it is possible to expand the options of the arrangement patterns of the sealed portion 50, and to make an arbitrary selection therefrom.

According to the first embodiment, it is possible to shape the edges constituting the first pressure-bonding parts 53 and the second pressure-bonding parts 54 with a curve, and to shape the auxiliary pressure-bonding parts 55 with an arbitrary shape such as a heart shape. The visibility is improved in the outer perimeter of the absorbent article 1 by alternately arranging the first pressure-bonding parts 53, the second pressure-bonding parts 54 and the auxiliary pressure-bonding parts 55 with such a shape. Particularly by forming with a curve, it is possible to provide the user with a softer impression than in a case of forming with a straight line.

In the first embodiment, the shape of the auxiliary pressure-bonding parts 55 is a heart shape or a circle, but it is not limited thereto. As the other shapes, it is possible to form with an arbitrary shape such as a triangle, a square, the marks of cards such as a spade shape, a star, and a flower shape.

The top sheet 20 is not limited to the first embodiment as long as it has the liquid permeability. For example, it is possible to use non-woven fabrics in which concavities and convexities are formed on the surface. In this case, the first pressure-bonding parts 53 or the second pressure-bonding parts 54 are formed in such a way to straddle the concavities and convexities formed on the surface of the non-woven fabrics with the concavities and convexities. As a result, though the peeling strengths of the pressure-bonding parts 51 are different depending on the difference of the weight of the concave portions and the convex portions of the top sheet 20, it is formed such that the peeling strength of the pressure-bonding parts 51 arranged on the convex portions is high. Thus, in cases where the top sheet portion 2 or the back surface sheet 30 peels off in the sealed portion 50, it is possible to prevent the peeling off at portions of the pressure-bonding parts 51 arranged on the convex portions that have high peeling strength, and to prevent the entire joined sheet from peeling off.

As the non-woven fabric on which concavities and convexities are formed, for example, it is possible to use a non-woven fabric in which a plurality of groove portions are formed with substantially equal intervals on one side of the non-woven fabric, and a plurality of convex portions are formed in each of the plurality of groove portions. It is preferable that a plurality of openings be formed with predetermined intervals in each of the plurality of groove portions. It is possible to form such a non-woven fabric which has a plurality of groove portions and a plurality of convex portions, and in which a plurality of openings are formed in each of the groove portions, by performing the following. For example, a fibrous web, which is a fiber aggregate, is supported from a bottom side by a netted supporting member having breathability, followed by blowing a gas from a top side, thereby moving fibers constituting the fibrous web. This fiber aggregate is like a sheet, and has a degree of freedom to which the fibers can move inside the fiber aggregate. Fiber orientation, fiber sparsity and density, or weight of the fibers is adjusted appropriately. Since the groove portions are formed on the top sheet 20, and the openings are provided in the groove portions, it is possible to obtain the absorbent article 1 with superior liquid permeability. Moreover, in cases where such a non-woven fabric is used as the top sheet 20, it is preferable to be used in such a way that the direction in which the groove portions extend coincide with the longitudinal direction LD of the absorbent article 1. By causing the direction in which the groove portions extend to coincide with the longitudinal direction LD of the absorbent article 1, it is possible to prevent the discharged bodily fluids from widely diffusing to the width direction WD of the absorbent article 1.

It should be noted that, in cases where fibers are arranged and rearranged by blowing an air stream, a fibrous web, which is formed by the card method that uses comparatively long fibers, is suitable. In order that after groove portions (concave and convex portions) are formed by a plurality of air flows, the fibers are made into a non-woven fabric with its shape retained, it is preferable to employ throw air method where thermoplastic fibers are subjected to hot melting by oven processing (heat treatment). Fibers suitable for this manufacture method are preferably those having the core-in-sheath structure or side-by-side structure in order to heat-seal intersecting points of the fibers or are more preferably those having the core-in-sheath structure in which sheaths can be easily heat-sealed in a secure manner. Particularly, a sheath-core composite fiber composed of polyethylene terephthalate and polyethylene, or a sheath-core composite fiber composed of polypropylene and polyethylene can be used suitably. These fibers can be used singly or in combination of two or more types. The fiber length is preferably 20 to 100 mm, especially 35 to 65 mm.

In the first embodiment, the pressure-bonding parts 51 are evenly formed in the sealed portion 50, but it is not limited thereto. Specifically, it is possible to form the pressure-bonding parts 51 more densely in a vicinity of a dislocation-preventing portion 31 or wing-side dislocation-preventing portions 32L and 32R than in the other portions. It is possible to partly increase the rigidity in the outer perimeter of the absorbent article 1 by partly increasing a unit area of the pressure-bonding parts 51 in the sealed portion 50. This makes it possible to prevent the portions with higher rigidity from twisting. It is possible to prevent the dislocation-preventing portion 31 or the wing-side dislocation-preventing portions 32L and 32R from sticking each other while in use, by arranging the pressure-bonding parts 51 densely in the vicinity of the dislocation-preventing portion 31 or the wing-side dislocation-preventing portions 32L and 32R.

In the first embodiment, compression grooves, which compress and join the second sheet 22 and an absorbent core 4, may be formed on the top sheet 20 in the absorbent article 1. For example, the compression grooves are linearly generated on the top sheet 20 by alternatingly forming high-compressed portions and low-compressed portions in series. Moreover, the compression grooves compress and join the each part, and the high-compressed portions form concave portions, thereby making it possible to prevent liquids such as the discharged matter from diffusing on the top sheet 20.

In the first embodiment, so-called gathers are not formed in the side sheets 21, but the gathers may be formed. Specifically, on the side of the top sheet 20, the fixing is carried out in a stretched stated with a predetermined stretching ratio with filar rubber or the like inside where it is folded in two. When the filar rubber shrinks to the original length, creases are created in the side sheets 21, and the gathers are formed. As a result, the side sheets 21 stand up three-dimensionally along the wearer's body, and act as leakage preventing walls in the top sheet 20.

In the first embodiment, wings 5L and 5R are formed, but it is not limited thereto, and the wings 5L and 5R may not be provided. It is possible to form the absorbent article 1 as an elongated shape in the longitudinal direction LD.

### 2. Other Embodiments

Other embodiments are explained below. The second to fifth embodiments are different from the first embodiment in the shape or arrangement of the pressure-bonding parts 51 in the sealed portion 50. It should be noted that, parts that are different from the first embodiment are mainly explained below. Moreover, the parts similar to those of the first embodiment are designated by the same numerals, and the parts without a particular description are similar to those of the first embodiment.

### 2-1. Second Embodiment

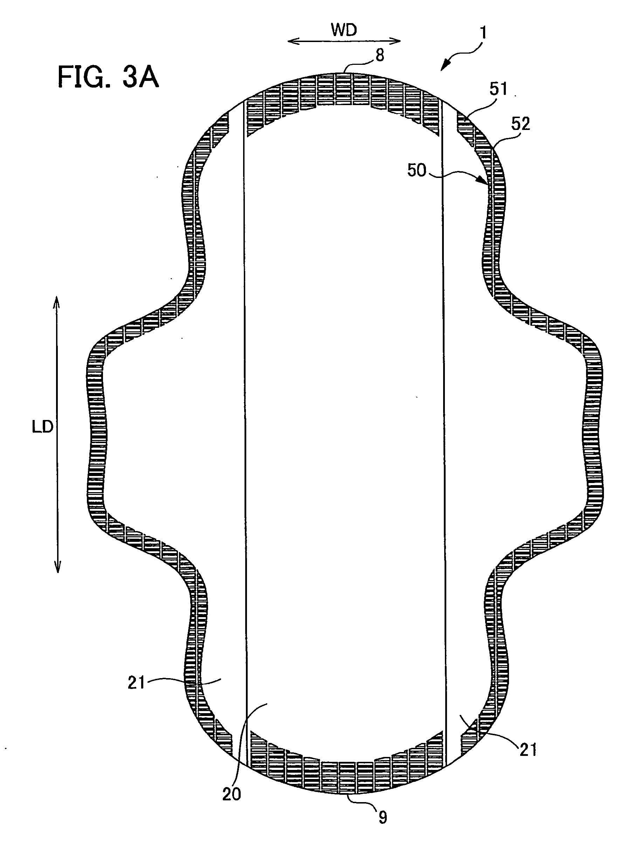

As shown in Fig. 3A, the absorbent article 1 in the second embodiment is different in the shape and arrangement pattern of the pressure-bonding parts 51 in the sealed portion 50 formed along the outer perimeter of the absorbent article 1.

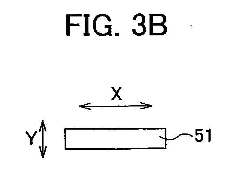

As shown in Figs. 3A and 3B, the pressure-bonding parts 51 of the second embodiment are arranged in such a way that all of the first directions X are in consonance with the width direction WD of the absorbent article 1. That is to say, the pressure-bonding parts 51 are arranged in such a way that edges extending in the first direction X are substantially orthogonal to the longitudinal direction LD of the absorbent article 1. The pressure-bonding parts 51 are rectangular, and the outer perimeter thereof is formed with straight lines. The pressure-bonding parts 51 adjacent to each other in the second direction Y are arranged in series to each other. In other words, the pressure-bonding parts 51 are arranged to line up at the same position as each other in the second direction Y. Thus, the non-pressure-bonding parts 52 are formed linearly and substantially in parallel to the longitudinal direction LD of the absorbent article 1.

Moreover, the pressure-bonding parts 51 adjacent to each other in the first direction X are also arranged to line up in the same position in the first direction X. Thus, the non-pressure-bonding parts 52 are, also in the first direction X, formed linearly and substantially in parallel to the width direction WD of the absorbent article 1.

The pressure-bonding parts 51 are formed in the entire outer perimeter of the absorbent article 1. The pressure-bonding parts 51 are arranged in such a way that the edges extending in the first direction X are substantially in parallel to the width direction WD of the absorbent article 1, in the sides along the longitudinal direction LD of the absorbent article 1, as well as in the regions in the vicinity of the front edge 8 and the rear edge 9.

Since the pressure-bonding parts 51 have a small length extending in the second direction Y, it is possible to widen the pitch between the pressure-bonding parts 51 adjacent to each other in the second direction Y, at the time of forming in the sealed portion 50. This makes it possible to reduce the rigidity of the region along the outer perimeter of the absorbent article 1, to maintain the feeling of the region, and to reduce the uncomfortability for the wearer.

On the other hand, edges extending in the second direction Y are formed substantially in parallel to the longitudinal direction LD, and short sides in the rectangular pressure-bonding parts 51 are arranged substantially in parallel to the longitudinal direction LD. This makes it possible to form the pressure-bonding parts 51 with a smaller pitch in the longitudinal direction LD, and to form the pressure-bonding parts 51, in a unit area, densely in the longitudinal direction LD.

As a result, the folding-starting points are increased in the longitudinal direction LD in the sealed portion 50 formed along the outer perimeter of the absorbent article 1, and if the entire absorbent article 1 is curved against the longitudinal direction LD, the outer perimeter can also be deformed along the curve of the wearer's body. The deformation along the wearer's body is possible, thereby eliminating a gap between the article and the wearer's body, making it possible to prevent the leakage.

### 2-2. Third Embodiment

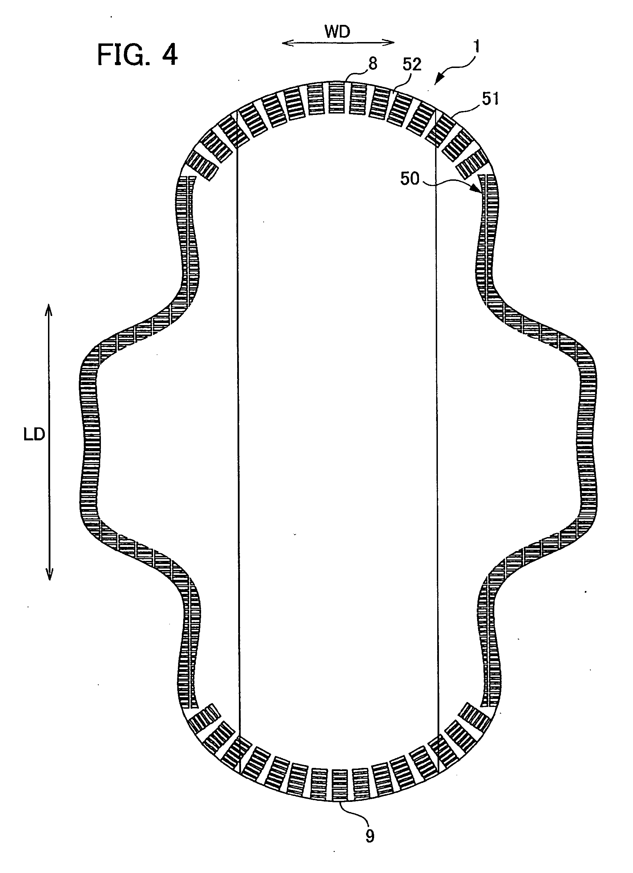

As shown in Fig. 4, the absorbent article 1 in the third embodiment is different in the shape and arrangement pattern of the pressure-bonding parts 51 in the sealed portion 50 formed along the outer perimeter of the absorbent article 1. It should be noted that the shape of the pressure-bonding parts 51 in the third embodiment is similar to the pressure-bonding parts 51 in the second embodiment. Moreover, the plurality of pressure-bonding parts 51 are formed with the substantially same shape.

In the sealed portion 50 formed in the side edges along the longitudinal direction LD of absorbent article 1 including the wings 5L and 5R, the pressure-bonding parts 51 are formed in such a way that the first direction X of the pressure-bonding parts 51 is substantially parallel to the width direction WD, as in the case of the second embodiment.

On the other hand, in the sealed portion 50 formed along the shape of the front edge 8 and the rear edge 9 in the absorbent article 1, the pressure-bonding parts 51 are formed in such a way that the edges extending in the first direction X thereof are along the circumference shape of the absorbent article 1. The pressure-bonding parts 51 adjacent to a predetermined pressure-bonding part 51 in the second direction Y are arranged in series to the predetermined pressure-bonding part 51. In other words, edges extending in the first direction X in the pressure-bonding parts 51 adjacent to each other in the second direction Y are arranged in parallel to each other, and edges extending in the second direction Y are arranged to intersect extension lines of edges extending in the other second other directions Y.

In this way, the first directions X in the pressure-bonding parts 51 are formed along the shape of the outer perimeter of the absorbent article 1, thereby the pressure-bonding parts 51 are not arranged on straight lines in the width direction WD in the sealed portion 50 in the vicinity of the front edge 8 and the rear edge 9. In other words, at the end to the side of the front edge 8 or the rear edge 9 in the absorbent article 1, the non-pressure-bonding parts 52 are not formed linearly in series in the width direction WD of the absorbent article 1. As a result, at the end to the side of the front edge 8 or the rear edge 9 in the absorbent article 1, resistance to the bending in the longitudinal direction LD is increased, thereby making the end hard to be the bending-starting point in the longitudinal direction LD. Thus, it is possible to suppress the uncomfortability such as pains caused by the folded corner abutting the wearer's body, the folding occurring at the end of the front edge 8 or the end of the rear edge 9 of the absorbent article 1 while in use.

### 2-3. Fourth Embodiment

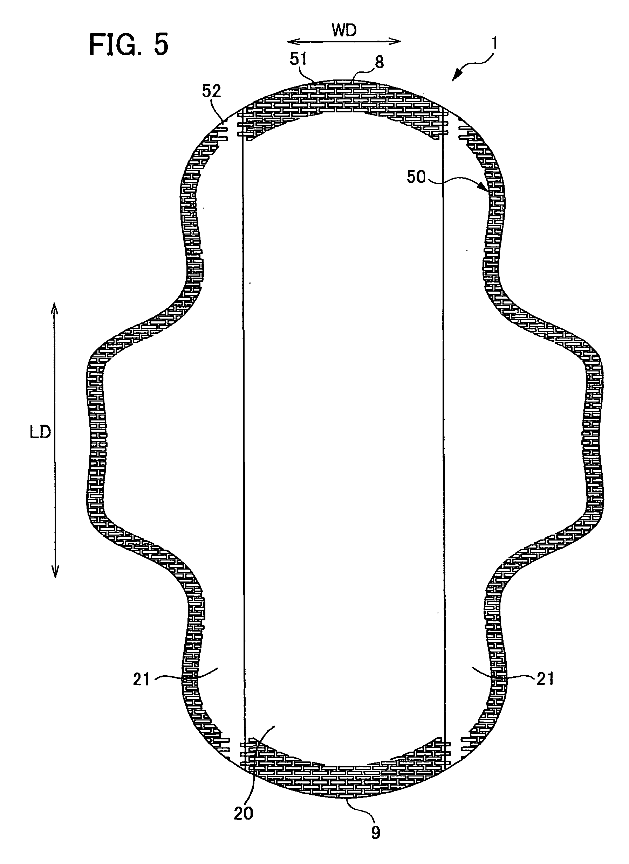

As shown in Fig. 5, the absorbent article 1 in the fourth embodiment is different in the shape and arrangement pattern of the pressure-bonding parts 51 in the sealed portion 50 formed along the outer perimeter of the absorbent article 1. It should be noted that the shape of the pressure-bonding parts 51 in the fourth embodiment is similar to the pressure-bonding parts 51 in the second embodiment. Moreover, the plurality of pressure-bonding parts 51 with the substantially same shape are formed in the sealed portion 50.

In the sealed portion 50 formed in the side edges along the longitudinal direction LD of absorbent article 1 including the wings 5L and 5R, the pressure-bonding parts 51 are formed in such a way that the first direction X is substantially parallel to the width direction WD, as in the case of the second embodiment.

Moreover, also in the sealed portion 50 formed in the vicinity of the front edge 8 and the rear edge 9 in the absorbent article 1, the pressure-bonding parts 51 are formed in such a way that the first direction X is substantially parallel to the width direction WD of the absorbent article 1.

Moreover, a predetermined pressure-bonding part 51 among the plurality of pressure-bonding parts 51 formed in the sealed portion 50 is arranged to intersect a straight line extending in the second direction Y from one end of the first direction X of the other pressure-bonding part 51 adjacent to each other in the second direction Y. Furthermore, a predetermined pressure-bonding part 51 among the plurality of pressure-bonding parts 51 does not intersect a straight line extending in the second direction Y from the other end of the first direction X of the other pressure-bonding part 51 adjacent to each other in the second direction Y. In other words, the plurality of pressure-bonding parts 51 are arranged in a relationship that an extension line of a side extending in the second direction Y in a predetermined pressure-bonding part 51 always intersects an edge extending in the first direction X in the other pressure-bonding part 51 adjacent to each other in the second direction Y. It should be noted that, a substantial center of a side extending in the first direction X of the other pressure-bonding part 51 is preferably arranged on the extension line of a side extending in the second direction Y in the predetermined pressure-bonding part 51.

Thus, the pressure-bonding parts 51 are not formed linearly in the longitudinal direction LD of the absorbent article 1. Moreover, the non-pressure-bonding parts 52 are not formed in series in the longitudinal direction LD. This prevents the absorbent article 1 from being folded in the width direction WD, and maintains the absorbent article 1 in a spread state, thereby preventing the twisting of the absorbent article 1 while in use. Moreover, since the non-pressure-bonding parts 52 are not formed in series in the longitudinal direction LD, a gap is eliminated in the direction of the bodily fluids to flow, particularly at the end of the front edge 8 and the rear edge 9 of the absorbent article 1, thereby making it possible to prevent the leakage.

### 2-4. Fifth Embodiment

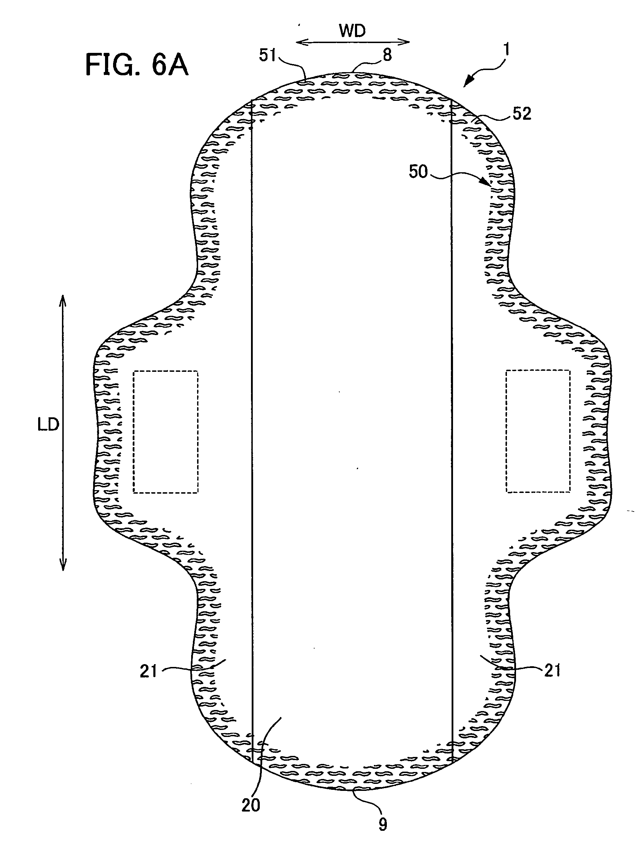

As shown in Fig. 6A, the absorbent article 1 in the fifth embodiment is different in the shape and arrangement pattern of the pressure-bonding parts 51 in the sealed portion 50 formed along the outer perimeter of the absorbent article 1.

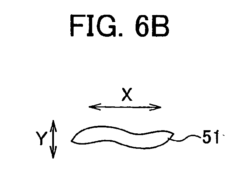

It should be noted that the shape of the pressure-bonding parts 51 in the fifth embodiment as shown in Fig. 6B is similar to the pressure-bonding parts 53 in the first embodiment. Moreover, the plurality of pressure-bonding parts 51 with the substantially same shape is formed in the sealed portion 50. Furthermore, the arrangement pattern of the pressure-bonding parts 51 is similar to the arrangement pattern of the pressure-bonding parts 51 in the fourth embodiment.

In the fifth embodiment, the shape of the pressure-bonding parts 51 is a shape in which all of the circumference forming the pressure-bonding parts 51 are constituted with a curve. Moreover, the non-pressure-bonding parts 52 are not formed linearly in series along the longitudinal direction LD of the absorbent article 1. In other words, the plurality of pressure-bonding parts 51 are arranged in such a way that, on an extension line of a side extending in the second direction Y of the pressure-bonding part 51, an edge extending in the first direction X in the other pressure-bonding part 51 adjacent to each other in the second direction Y is arranged.

In this embodiment, since the shape of the pressure-bonding parts 51 is constituted with a curve, there is not a region constituted with a straight line in the sealed portion 50. Thus, it is possible to disperse the folding-starting points at which the absorbent article 1 bends in the longitudinal direction LD, and to improve the feel of the sealed portion 50. Furthermore, it is possible to prevent the sealed portion 50 from being bent, particularly in the vicinity of the front edge 8 or the rear edge 9, while in use of the absorbent article 1.

## Claims

1. An elongated absorbent article (1), comprising
a top sheet portion (2) having a sheet, at least a part of which is liquid-permeable;
a back surface sheet portion (3) having a liquid-impermeable sheet; and a liquid retentive absorbent core (4) arranged between the top sheet portion (2) and the back surface sheet portion (3),
wherein a joined region (50), which joins the back surface sheet portion (3) and the top sheet portion (2), is formed in an outer perimeter of the absorbent article (1),
wherein a plurality of pressure-bonding parts (51) are formed in all or part of the joined region (50), and
wherein each of the plurality of pressure-bonding parts (51) is formed so that the length in a first direction (X) is longer than the length in a second direction (Y) that is orthogonal to the first direction (X),
wherein all or part of the plurality of pressure-bonding parts (51) formed at the ends of a longitudinal direction of the absorbent article (1) is formed to be substantially orthogonal to the longitudinal direction.

2. The absorbent article according to claim 1,
wherein the plurality of pressure-bonding parts (51) have first pressure-bonding parts (53) and second pressure-bonding parts (54), and wherein the second pressure-bonding parts (54) are formed in the second direction (Y) in the first pressure-bonding parts (53).

3. The absorbent article according to any one of claims 1 to 2, wherein, in all or part of the plurality of pressure-bonding parts (51), the first direction (X) is formed to be substantially parallel to the outer perimeter.

4. The absorbent article according to any one of claims 2 to 3,
wherein the second pressure-bonding part (54) is formed to intersect a straight line extending in the second direction (Y) from one end of the first direction (X) of the first pressure-bonding part (53), and not to intersect a straight line extending in the second direction (Y) from the other end of the first pressure-bonding part (53) in the first direction (X).

5. The absorbent article according to any one of claims 2 to 4,
wherein the plurality of first pressure-bonding parts (53) and second pressure-bonding parts (54) are respectively formed, wherein the plurality of first pressure-bonding parts (53) and the plurality of second pressure-bonding parts (54) are formed in such a way that the first direction (X) is substantially parallel to the outer perimeter, and are formed in series in the outer perimeter direction, and wherein non-pressure-bonding regions (52) formed between the plurality of first pressure-bonding parts (53) and the plurality of second pressure-bonding parts (54) are formed to be substantially parallel to the outer perimeter direction, and are formed to extend in the outer perimeter direction.

6. The absorbent article according to any one of claims 1 to 5,
wherein, in a predetermined position in the outer perimeter, the joined region (50) is not formed or a region is formed in which the number of the plurality of pressure-bonding parts (51) is small.

## Patentansprüche

1. Länglicher absorbierender Artikel (1), enthaltend
einen Decklagenabschnitt (2), der zumindest in einem Teil flüssigkeitsdurchlässig ist;
einen Außenflächenlagenabschnitt (3), der eine flüssigkeitsundurchlässige Lage hat; und
einen Flüssigkeit zurückhaltenden absorbierenden Kern (4), der zwischen dem Decklagenabschnitt (2) und dem Außenflächenlagenabschnitt (3) angeordnet ist,
wobei ein Verbindungsbereich (50), der den Außenflächenlagenabschnitt (3) und den Decklagenabschnitt (2) verbindet, in einem äußeren Umfang des absorbierenden Artikels (1) gebildet ist,
wobei eine Vielzahl von Druckverbindungsteilen (51) in dem gesamten oder einem Teil des Verbindungsbereichs (50) gebildet sind, und
wobei jeder der Vielzahl von Druckverbindungstellen (51) so gebildet ist, dass die Länge in einer ersten Richtung (X) länger ist als die Länge in einer zweiten Richtung (Y), die senkrecht zu der ersten Richtung (X) ist,
wobei alle oder ein Teil der Vielzahl von Druckverbindungsteilen (51), die an den Enden einer Längsrichtung des absorbierenden Artikels (1) gebildet sind, so gebildet sind, dass sie Im Wesentlichen senkrecht zur Längsrichtung sind.

2. Absorbierender Artikel nach Anspruch 1,
bei welchem die Vielzahl von Druckverbindungsteilen (51) erste Druckverbindungstelle (53) und zweite Druckverbindungsteile (54) aufweist und bei welchem die zweiten Druckverbindungstelle (54) In der zweiten Richtung (Y) in den ersten Druckverbindungsteilen (53) gebildet sind.

3. Absorbierender Artikel nach einem der Ansprüche 1 bis 2, bei welchem bei allen oder einem Teil der Vielzahl von Druckverbindungsteilen (51) die erste Richtung (X) Im Wesentlichen parallel zu dem äußeren Umfang gebildet ist.

4. Absorbierender Artikel nach einem der Ansprüche 2 bis 3,
bei welchem der zweite Druckverbindungsteil (54) so gebildet Ist, dass er eine gerade Linie schneidet, die in der zweiten Richtung (Y) von einem Ende der ersten Richtung (X) des ersten Druckverbindungsteils (53) verläuft, und eine gerade Linle nicht schneidet, die in der zweiten Richtung (Y) von dem anderen Ende des ersten Druckverbindungstells (53) In der ersten Richtung (X) verläuft.

5. Absorblerender Artikel nach einem der Ansprüche 2 bis 4,
bei welchem die Vielzahl der ersten Druckverbindungstelle (53) und zweiten Druckverbindungsteile (54) jeweils gebildet ist, wobei die Vielzahl der ersten Druckverbindungsteile (53) und die Vielzahl der zweiten Druckverbindungsteile (54) dergestalt gebildet Ist, dass die erste Richtung (X) im Wesentlichen parallel zu dem äußeren Umfang ist, und in Reihe In Richtung des äußeren Umfangs gebildet sind, und wobei zwischen der Vielzahl der ersten Druckverbindungsteile (53) und der Vielzahl der zweiten Druckverbindungsteile (54) gebildete Nicht-Druckverbindungsteile (52) so gebildet sind, dass sie im Wesentlichen parallel zu der Richtung des äußeren Umfangs sind, und so gebildet sind, dass sie in der Richtung des äußeren Umfangs verlaufen.

6. Absorbierender Artikel nach einem der Ansprüche 1 bis 5,
bei welchem an einer vorbestimmten Position in dem äußeren Umfang der Verbindungsbereich (50) nicht gebildet ist oder ein Bereich gebildet ist, in welchem die Anzahl der Vielzahl von Druckverbindungsteilen (51) klein ist.

## Revendications

1. Article absorbant allongé (1), comprenant
une partie de feuille supérieure (2) ayant une feuille, dont au moins une partie est perméable aux liquides;
une partie de surface arrière de feuille (3) comportant une feuille imperméable aux liquides, et
un noyau absorbant de rétention de liquide (4) disposé entre la partie de feuille supérieure (2) et la partie de surface arrière de feuille (3),
dans lequel une région de liaison (50), qui relie la partie de surface arrière de feuille (3) et la partie de feuille supérieure (2), est formée dans un périmètre extérieur de l'article absorbant (1),
dans lequel plusieurs parties de liaison (51) par pression sont formées dans toute ou une partie de région de liaison, et
dans lequel chacune des plusieurs parties de liaison (51) par pression est formée de telle sorte que la longueur dans une première direction (X) est plus longue que la longueur dans une deuxième direction (Y) qui est orthogonale à la première direction (X),
dans lequel toute ou une partie de la pluralité de parties de liaison (51) par pression, formée aux extrémités d'une direction longitudinale de l'article absorbant (1), est formée pour être essentiellement orthogonale à la direction longitudinale.

2. Article absorbant selon la revendication 1,
dans lequel les plusieurs parties de liaison (51) par pression ont des premières parties de liaison (53) par pression et des deuxièmes parties de liaison (54) par pression, et dans lequel les deuxièmes parties de liaison (54) par pression sont formées dans la seconde direction (Y) dans les premières parties de liaison (53) par pression.

3. Article absorbant selon l'une quelconque des revendications 1 à 2, dans lequel dans la totalité ou une partie de la pluralité des parties de liaison (51) par pression, la première direction (X) est formée pour être sensiblement parallèle au périmètre extérieur.

4. Article absorbant selon l'une quelconque des revendications 2 à 3, dans lequel la seconde partie de liaison (54) par pression est formée de manière à croiser une ligne droite s'étendant dans la seconde direction (Y) à partir d'une extrémité de la première direction (X) de la première partie de liaison (53) par pression, et pour ne pas croiser une ligne droite s'étendant dans la seconde direction (Y) à partir de l'autre extrémité de première partie de liaison (53) par pression dans la première direction(X).

5. Article absorbant selon l'une quelconque des revendications 2 à 4,
dans lequel la pluralité de premières parties de liaison (53) par pression et de deuxièmes parties de liaison (54) par pression sont respectivement formées, dans lequel la pluralité de premières parties de liaison (53) par pression et la pluralité de deuxièmes parties de liaison (54) par pression sont formées de telle sorte que la première direction (X) est sensiblement parallèle au périmètre extérieur, et sont formées en série dans la direction du périmètre extérieur, et dans lequel les régions (52) de non liaison par pression formées entre la pluralité de premières parties de liaison (53) par pression et la pluralité de deuxièmes parties de liaison (54) par pression sont formées pour être sensiblement parallèles à la direction du périmètre extérieur, et sont formées pour s'étendre dans la direction de périmètre extérieur.

6. Article absorbant selon l'une quelconque des revendications 1 à 5,
dans lequel, dans une position prédéterminée dans le périmètre extérieur, la région de liaison (50) n'est pas formée ou une région est formée dans laquelle le nombre de la pluralité de parties de liaison (51) à la pression est petit.
